# EUROPÄISCHE PATENTSCHRIFT

(11) **EP 0 849 572 B1**
(45) Veröffentlichungstag und Bekanntmachung des Hinweises auf die Patenterteilung: **19.03.2003**
(21) Anmeldenummer: 97250372.6
(22) Anmeldetag: 15.12.1997
(51) Int. Cl.: G01G 17/00

(54) **Verfahren und Vorrichtung zur Kontolle der Tablettenparameter**
Process and apparatus for controlling tablet parameters
Procédé et dispositif de contrôle des paramètres des comprimés

(30) Priorität: 20.12.1996 DE 19654612
(43) Veröffentlichungstag der Anmeldung: 24.06.1998
(73) Patentinhaber: KORSCH PRESSEN GmbH, D-13509 Berlin (DE)
(72) Erfinder: Fiedler, Jürgen, 13505 Berlin (DE); Körner, Hans Georg, 15236 Frankfurt (Oder) (DE); Hegel, Walter, 13437 Berlin (DE); Wagner, Udo, 15848 Tauche (DE); Bargenda, Hagen, 15234 Frankfurt (Oder) (DE); Stepanek, Josef, 15234 Frankfurt (Oder) (DE)
(74) Vertreter: Hengelhaupt, Jürgen, Dipl.-Ing.

(56) Entgegenhaltungen:
- EP-A- 0 045 319
- EP-A- 0 181 946
- CH-A- 657 920
- DE-U- 8 402 581
- US-A- 4 884 463

## Beschreibung

Die Erfindung bezieht sich auf ein Verfahren zur Kontrolle der Tablettenparameter Gewicht, Höhe und Härte mittels Wägen und Messen, und auf eine Vorrichtung zur Durchführung des Verfahrens als Einzelgerät und vorzugsweise für eine Rundlauf-Tablettenpresse gemäß dem Oberbegriff des Anspruches 1 bzw.des Anspruches 2.

Ein wichtiger Qualitätsparameter für die Tablettenherstellung ist die Einhaltung des vorgegebenen Tablettengewichts. Grundlage für jede automatische Steuerung einer Tablettiermaschine ist der Zusammenhang zwischen gemessener maximaler Preßkraft einer Tablette und der Masse des in die Matrize gefüllten Gutes (Tablettengewicht) unter der Voraussetzung gleicher Materialdaten (Schüttgewicht). Es besteht dabei ein direkter Zusammenhang zwischen dem Tablettengewicht und der für die Fertigung der kontrollierten Tablette notwendigen Preßkraft. Dieser Zusammenhang wird zum Einsatz von automatischen Überwachungsund Steuergeräten an Rundlaufpressen ausgenutzt. In Abhängigkeit vom zu verpressenden Material ist jedem Tablettengewicht bei einer durch die Preßwerkzeuge vorgegebenen Tablettenform und einer eingestellten Tablettenhöhe eine bestimmte Preßkraft zugeordnet. Schwankt bei konstanter Tablettenhöhe die Füllmenge und damit das Tablettengewicht, resultiert daraus in direkter Abhängigkeit eine Preßkraftänderung.

An modernen Rundlauf-Tablettenpressen, die viele Einzelpreßformen auf einer Kreisbahn enthalten und eine große Anzahl von Tabletten pro Sekunde pressen, sind die Parameter Gewicht, Höhe und Härte mittels spezifischer Betriebsparameter für jede Einzelpreßform mehr oder weniger direkt einstellbar.

Das Verhältnis der spezifischen Betriebsparameter der Einzelpreßform zum erreichten Tablettenparameter muß jedoch wegen der Eigenschaftsvielfalt der zu verpressenden Massen kontinuierlich kontrolliert und geregelt werden. Dabei muß die eindeutige Zuordnung der real erreichten Tablettenparameter zu den Betriebsparametern der Einzelpreßform sichergestellt sein.

Es sind bereits Vorrichtungen bekannt, mit denen Einzelprüfungen der Parameter Gewicht, Höhe und Härte an einer Tablette vorgenommen werden können.

Bei Pharmacheck 1 (Bedienungsanleitung Pharmakontroll 3 NET, Kapitel 3, Seiten 11, 16 bis 18, der Korsch Pressen GmbH, 1992/1993) werden einzelne Tabletten aus der Tablettenpresse aussortiert und in einer Waage gewogen. Nach dem Wiegen der ersten Tablette wird die Waage auf Null gestellt und eine zweite Tablette wird gewogen. Wenn 10 oder 20 Tabletten in der Waagschale sind, wird diese entleert, wobei die Tabletten über drei verschiedene Auslaßkanäle je nach Qualität abgeführt werden. Statt der Einzelwägung kann eine Charge von 10 oder 20 Tabletten gleichzeitig der Tablettenpresse entnommen und gemeinsam gewogen werden. Mit den Wägungen wird das Gewicht der einzelnen Tabletten bis auf eine Genauigkeit von 0,1 mg ermittelt. Das Wägeergebnis wird zur Steuerung des Füllungsgrades der einzelnen Matrizen verwendet.

Bei Pharmacheck 2 (Bedienungsanleitung Pharmakontroll 3 NET, Kapitel 4, Seiten 5,9,10, 12 bis 16, der Korsch Pressen GmbH, 1992/1993) werden einzelne Tabletten dem um eine vertikale Achse drehbaren Drehstern zugeführt, wobei durch eine Drehung des Drehsternes die einzelnen Tabletten nacheinander einer Wägeeinheit zur Feststellung des Gewichtes, einer Meßeinheit zur Feststellung von Höhe bzw. Dicke und einer weiteren Meßeinheit zur Feststellung von Durchmesser und Härte und anschliessend einer Auswerfeinheit zugeführt werden.Die Taktung erfordert einen Zeittakt von mindestens 6 Sekunden pro Tablette und pro Station, so daß bei einer Tablettenpresse mit einer Arbeitsgeschwindigkeit von 10.000 Stück pro Minute nur eine geringe Zahl von Tabletten geprüft werden kann.

Nachteilig bei den bekannten Lösungen ist es außerdem, daß die Durchführung von Einzelprüfungen keine produktive Meßdurchführung an einer Vielzahl von Tabletten und keine präzise Prozeßdurchführung auf der Basis von einzeln bestimmten Tablettenparametern, speziell dem Tablettengewicht zuläßt.Der Ausschußanteil ist dadurch in der Tablettenfertigung noch unbefriedigend hoch. Es ist praktisch unmöglich, bei der Wägung von einzelnen Tabletten auf einen gesicherten Datenbestand zurückzugreifen, der eine gesicherte Erkenntnis ermöglicht,daß. das gewogene Gewicht der einzelnen Tablette dem der insgesamt produzierten Tabletten entspricht. Erst dann sind die Parameter für Höhe und Härte zu ermitteln.

Die Bestimmung von Gewicht, Höhe und Härte an jeweils einer Tablette nacheinander ist nicht ausreichend zuverlässig der die Prüftablette erzeugenden Preßform zuordenbar, so daß durchgeführte Korrekturen an der Tablettenpresse bei festgestellten Abweichungen oft wirkungslos bleiben.

Beim Sortenwechsel ist ein erheblicher Reinigungsaufwand der Prüfvorrichtung erforderlich, da sich der staubförmige Abrieb beim Verpressen und Transport der Tabletten in der gesamten Vorrichtung verteilt. Die Prüfergebnisse werden dadurch zum Teil verfälscht.

Im DE-GM 94 06 712 ist eine Vorrichtung zum Entgraten und Entstauben von Tabletten mit Saugluft beschrieben, bei der die Tabletten durch den Sog der Absauganlage über eine Blechrinne zwischen der Tablettenaufgabe und Tablettenabgabe gefördert und dabei entgratet und entstaubt werden. Diese Lösung ist für die nachfolgenden Prüfschritte an den Tabletten nicht geeignet.

Zur Durchführung der Höhen- und Härtemessungen müssen die Tabletten in der Prüfvorrichtung so abgelegt werden, daß auch Tabletten mit annähernd gleichen Maßen für Dicke, Länge und Durchmesser zuverlässig vermessen werden können. Die bekannten Einrichtungen hierzu sind unbefriedigend und verhindern nur unzureichend entsprechende Fehlmessungen.

Der Erfindung liegt die Aufgabe zugrunde, diese Nachteile zu vermeiden und das Verfahren und die Vorrichtung zur Kontrolle der Tablettenparameter zu verbessern.

Die Lösung dieser Aufgabe ergibt sich aus den Merkmalen der Patentansprüche 1 und 2. Damit sind eine schnelle, zuverlässige, reproduzierbare und verfälschungssichere Prüfung wahlweise aller Parameter oder ausgewählter Parameter an einer Tablette und die Summenprüfung des Gewichts an einer Mehrzahl von Tabletten gewährleistet.

Das erfindungsgemäße Verfahren und die erfindungsgemäße Vorrichtung gewährleisten durch die Prüfabfolge einer Kombination von Einzel- und/oder von Summenprüfungen in einem System eine dauerhafte Einhaltung der vorgegebenen Tablettenparameter während der Fertigung sowie eine hohe Zuordnungssicherheit der Meßwerte an einer Tablette zu den sie erzeugenden Preßformen.

Es kann ereignisabhängig, preßortabhängig oder frei definiert werden, wie eine Folge von Einzel- und/oder von Summenprüfungen von Tabletten erfolgen sollen.

Es sind wahlweise vollautomatisch eine Mehrzahl von Tabletten auf den Wägeaufsatz zu- und abführbar und ein Summengewicht sowie ein gemitteltes Einzelgewicht oder ein Einzelgewicht einer Tablette bestimmbar.

Durch den selbstreinigenden Tablettentransport sowie eine Selbstreinigung der Prüfbaugruppen wird eine Prozeßreinheit in der gesamten Vorrichtung erreicht.

Während der Höhen- und Härtemessung an der Tablette ist eine eindeutige Orientierung aller Tablettengeometrien gesichert.

Es können durch den modularen Aufbau der Vorrichtung mit geringem Aufwand jederzeit Veränderungen vorgenommen werden, um die Vorrichtung an die herrschenden Bedürfnisse anzupassen.

Der Austausch von Baugruppen, z.B. der Rinne in der Härte-Prüfstation zur Orientierung der Tabletten in Bruchkraftrichtung, erfolgt werkzeuglos mit wenigen Handgriffen.

Die Höhen- und die Gewichtsmessungen erfolgen für alle Tablettenformen und Tablettenabmessungen ohne Austausch von Vorrichtungselementen.

Es wird eine wesentliche Erhöhung der pro Zeiteinheit prüfbaren Tabletten erreicht.

Weitere vorteilhafte Ausgestaltungen der Erfindung ergeben sich aus den Unteransprüchen.

Die Erfindung ist nachfolgend anhand eines in der Zeichnung dargestellten Ausführungsbeispieles der Vorrichtung zur Kontrolle der Tablettenparameter näher erläutert. Es zeigen :
- Fig. 1: die schematische Darstellung einer Summengewichtsprüfung von Tabletten,
- Fig. 2: die schematische Darstellung der Einzelgewichtsprüfung von Tabletten,
- Fig. 3: die schematische Darstellung des Tablettentransports von der Wägezur Höhenprüfungs-Station,
- Fig. 4: die schematische Darstellung der Rinne mit Abstreifer, Schaber und Brechbacken,
- Fig. 5: den Schnitt V-V in Fig. 4c und
- Fig. 6: die schematische Darstellung der Schaberfunktion.

Die Vorrichtung zur Kontrolle der Tablettenparameter Gewicht, Höhe und Härte ist als Einzelgerät oder in einer steuerungstechnischen und/oder mechanischen Verbindung mit einer Tablettenpresse, vorzugsweise eine Rundlauf-Tablettenpresse, einsetzbar.

Die in ihren Parametern zu prüfenden Tabletten werden aus einer nicht dargestellten Tablettenpresse nach einem vorgegebenen Zyklus über einen Zuführkanal in die Vorrichtung eingebracht und nach Maßgabe eines vorgegebenen Prüfprogramms geprüft.

Die Auswahl der Tabletten kann jeweils von gleichen oder von unterschiedlichen, vorwählbaren Stempelstationen erfolgen. An jeder Einzeltablette können alle oder ausgewählte Parameter geprüft werden. An einer Menge von Tabletten kann das Gewicht der Tabletten ermittelt werden.

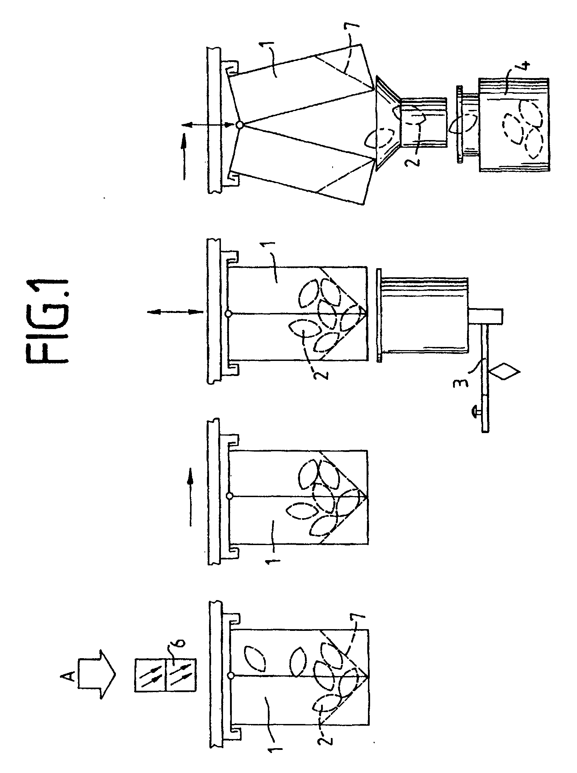

In der Fig. 1 ist in einer schematischen Darstellung gezeigt, in welchen Schritten eine Summengewichtsprüfung an einer definierten Anzahl von Tabletten 2, z.B. 100 Stück, durchgeführt wird.

Vom Ausgang A einer nicht dargestellten Tablettenpresse wird eine definierte Anzahl von Tabletten 2 über eine Zähleinrichtung 6, z.B. zwei Lichtschranken, in einen Aufnahmebehälter 1 gefüllt. Der Aufnahmebehälter 1 ist entlang seiner Längsachse geteilt und aufklappbar und weist in seinem unteren Bereich zur Bodenmitte bzw. zur Mitte der Ausfallöffnung geneigte Schrägen 7 auf. Der mit den Tabletten 2 gefüllte Aufnahmebehälter 1 wird zu einer Wägestation 3 transportiert und dort zusammen mit den Tabletten 2 gewogen. Der Meßwert wird in einer nicht dargestellten elektronischen Verarbeitungseinheit ausgewertet. Nach der Wägung wird der Aufnahmebehälter 1 in eine Entleerungsposition gefahren, dort werden die Tabletten 2 in einen Auffangbehälter 4 geschüttet, wozu der Aufnahmebehälter 1 aufgeklappt wird und die Tabletten 2 über die Schrägen 7 herausrutschen. Der Aufnahmebehälter 1 wird danach wieder in seine Ausgangsposition gefahren und kann erneut gefüllt werden.

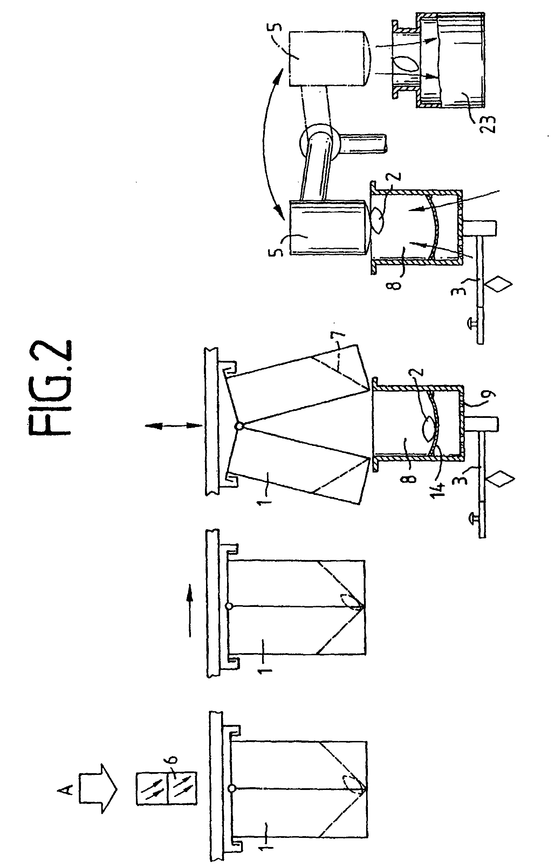

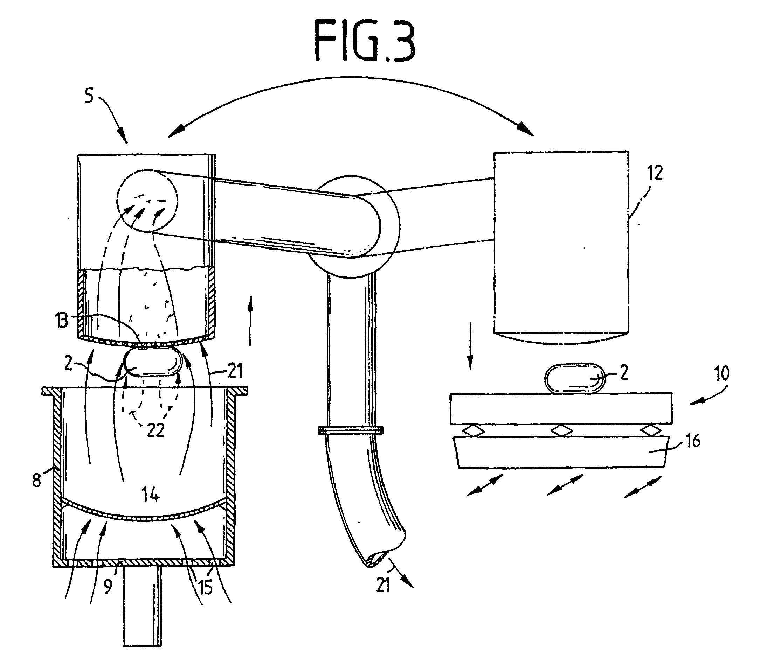

In der Fig. 2 ist in einer schematischen Darstellung gezeigt, wie mit demselben Aufnahmebehälter 1 eine Einzelgewichtsprüfung durchgeführt wird. Am Ausgang A der Tablettenpresse wird über die Zähleinrichtung 6 eine einzige Tablette 2 in den Aufnahmebehälter 1 gefüllt. Der Aufnahmebehälter 1 wird zur Wägestation 3 gefahren und dort durch Aufklappen entleert. Die Tablette 2 fällt auf einen nach unten konkav gewölbten Siebeinsatz 14 oberhalb eines mit Schlitzen 15 versehenen Wägeaufsatzbodens 9 eines Wägeaufsatzes 8 (Fig. 3) und wird dort gewogen. Der Wägeaufsatz 8 ist so gestaltet, daß die horizontalen Flächen minimiert sind, um die Zuganfälligkeit der Wägung zu verringern und um eine hohe Genauigkeit bei kurzer Taktzeit der Wägung zu gewährleisten.Der Meßwert wird entsprechend dem vorgegebenen Programm verarbeitet. Die Einzelgewichtsprüfung wird beendet, indem die Tablette 2 mittels eines Saugheberarms 5 (Fig. 2) aus dem Wägeaufsatz 8 entnommen und in den Restebehälter 23 befördert wird.

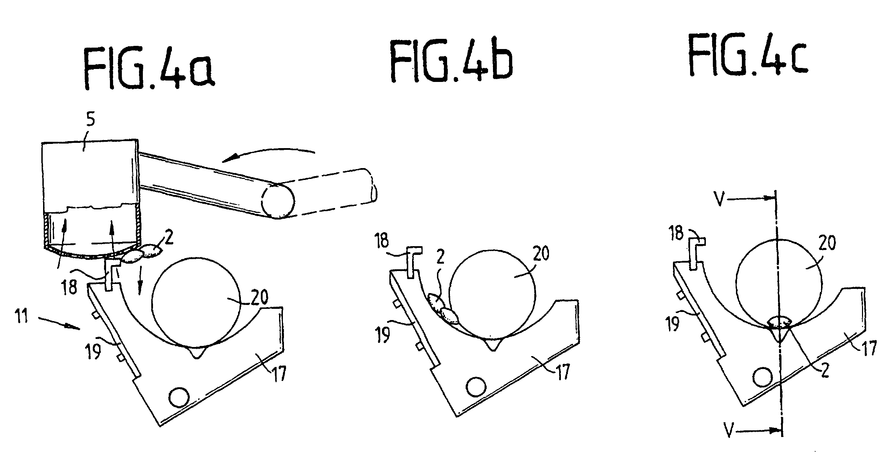

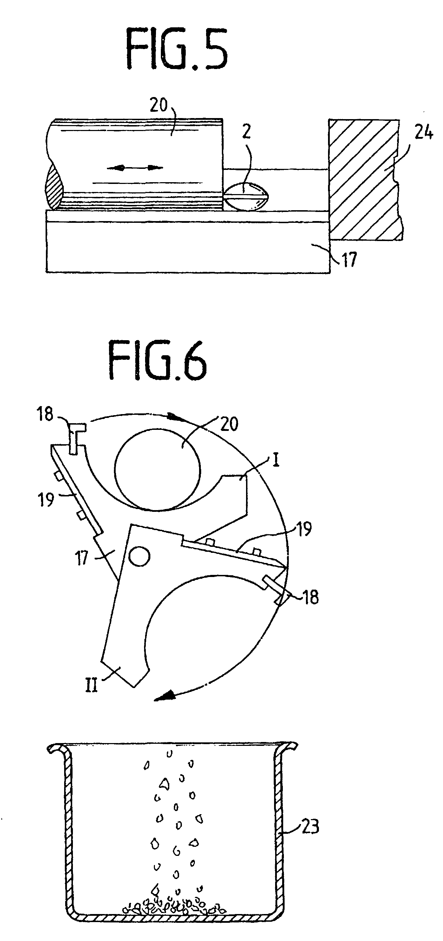

Wenn das Prüfprogramm weitere Prüfungen der entnommenen Tablette 2 vorsieht, dann wird die Tablette 2 anstelle in das Gefäß 4 zu einer weiteren Prüfstation, zum Beispiel einer Höhenmeß-Station 10 (Fig. 3) oder einer Härte-Prüfstation 11 (Fig. 4,5) mittels des Saugheberarms 5 transportiert und dort der Höhen- oder Härteprüfung unterzogen.

In der Fig. 3 sind die spezielle Ausführung des Saugheberarms 5 und die spezielle Ausführung des Wägeaufsatzes 8 schematisch dargestellt.

Der Saugheberarm 5 nach Fig. 3 wird für den Tablettentransport zwischen den Prüfstationen für die Gewichtsmessung 3, die Höhen- bzw. Dickenmessung 10 und die Härtemessung 11 verwendet und gewährleistet die Prozeßreinheit bei der Meßwertbildung, indem kein Abrieb entsteht und vorhandene Partikel abgesaugt und von den Meßvorgängen ferngehalten werden.

Der Saugheberarm 5 wird entsprechend der Fig. 3 im wesentlichen aus einem über ein nicht näher ausgeführtes Gelenksystem beweglichen Saugkopf 12 mit einem konvex nach außen gekrümmten Siebboden 13 gebildet, durch den Luft 21 über einen schaltbaren Seitenkanalverdichter gesaugt wird.Der Saugheberarm 5 ist so ausgestaltet, daß er zu den Stationen 3,10,11 geschwenkt werden kann. Der konvex gekrümmte Siebboden 13 gewährleistet, daß die Tabletten 2 unabhängig von ihrer Form in der Mitte des Siebbodens 13 anliegen.

Zur sicheren Funktionsfähigkeit des Saugheberarms 5 ist es wichtig, daß der Wägeaufsatzboden 9 des Wägeaufsatzes 8 Schlitze 15 definierter Größe aufweist, damit ein ausreichender und in der Strömungsgeschwindigkeit vorausbestimmter Luftstrom 21 durch den Siebboden 13 des Saugheberarms 5 fließen kann. Der Wägeaufsatz 8 enthält den nach unten konkav gekrümmten Siebeinsatz 14, um die aufgesetzten Tabletten 2 in eine stabile Lage zu bringen, die Tabletten 2 fallen durch die Krümmung des Siebeinsatzes 14 um.

Der Luftstrom 21 gewährleistet den Abtransport aller Abriebteile 22 und sonstigen Partikel, die die Messungen verfälschen können, in ein nicht gezeigtes Filter.

Entsprechend der Darstellung in der Fig. 3 wird die Tablette 2 durch den Luftstrom 21 getragen und am Siebboden 13 des Saugkopfes 12 festgehalten und durch Verschwenken des Saugheberarmes 5 in eine Position über der Prüfstation 10 zur Höhenmessung gebracht und dort durch Abschalten des Luftstromes 21 abgelegt. Eine Rüttelvorrichtung 16 unterhalb der Tablettenablage bringt die Tablette 2 in die stabile Schwerpunktlage, so daß gewährleistet ist, daß die Tablette 2 unabhängig von ihrer Form in der Höhe bzw. Dicke nach den bekannten Verfahren vermessen wird. Die richtige Lage der Tablette 2 ist insbesondere dann schwierig herstellbar gewesen, wenn das Dickenmaß nur um einen geringen Faktor kleiner ist als das zweite Raummaß.

Nach dem Vermessen der Höhe wird die Tablette 2 mittels des Saugheberarms 5 zur Prüfstation 11 zur Bestimmung der Härte transportiert (Fig. 4).

Die Messung der Härte der Tablette ist eine Bruchkraftbestimmung. Die Tablette wird zwischen einer beweglichen Brechbacke 20 und einer festen Meßbacke 24 (Fig. 5) positioniert. Die bewegliche Brechbacke 20 fährt so lange gegen die feste Meßbacke 24, bis die ansteigende Preßkraft abrupt abfällt. Die in diesem Moment erreichte Kraft wird als Bruchkraft bzw. als Tablettenhärte gemessen.

Entsprechend der Darstellung in Fig. 4 bis 6 weist die Prüfstation 11 zur Härte- bzw. Bruchfestigkeitsprüfung eine Rinne 17 zwischen der Brechbacke 20 und der Meßbacke 24 auf, die zusammen mit dem Backenvorschub die Längs- und Mittenausrichtung der verschieden geformten Tabletten 2 zur Bruchkraftrichtung vornimmt. An der Rinne 17 sind ein Abstreifer 18 und ein Schaber 19 angeordnet.

Mittels des Abstreifers 18 wird entsprechend den Darstellungen in der Fig. 4 beim Ablegen der Tablette 2 in die Rinne 17 der Prüfstation 11 eine Vororientierung der Tabletten 2 erreicht. Der Saugheberarm 5 wird beim Absetzen der Tablette 2 auf die Prüfstation 11 so positioniert (Fig. 4a), daß der Abstreifer 18 die Tablette 2 in eine vororientierte Lage bringt (Fig. 4b) und die Tablette 2 infolge der geometrischen Gestaltung der Rinne 17 in die endgültige Lage gleitet (Fig. 4c). Die Rinne 17 ist der Geometrie der Tabletten 2 angepaßt, so daß auch die schwierig zu handelnden Oblong-Tabletten richtig geführt und der Härteprüfung unterzogen werden können. Während die Höhen- und die Gewichtsbestimmung für alle Tablettengeometrien mit einer Art von Vorrichtungselementen geprüft werden, wird bei der Härteprüfung die Rinne 17 entsprechend der Tablettengeometrie ausgetauscht.

Die Rinne 17 weist vorzugsweise eine Querschnittsgeometrie in Form einer Klammer auf.

Mit der horizontalen Bewegung der beweglichen Brechbacke 20 (Fig.5) wird die Tablette 2 gegen einen feststehenden Teil der Rinne 17 geschoben, der die feststehende Meßbacke 24 bildet. Dabei wird die Tablette 2 orientiert 4) und letztlich zerstört. Die dabei entstehende Bruchkraft wird gemessen und im System weiterverarbeitet. Die Brechbacke 20 fährt dann in die Ausgangsposition zurück.

In der Fig. 6 ist die Funktion des Schabers 19 an der Rinne 17 dargestellt. Die Meß- bzw. Brechbacken 20 bzw. 24 der Härtemessung werden nach jedem Prüfvorgang mittels des Schabers 19 gereinigt. Aus der Ruhe-Position I wird die Rinne 17 mit dem Schaber 18 in die Arbeits-Position II bewegt und dabei an der Brech- bzw. Meßbacke 20 bzw. 24 entlanggeführt, so daß eventuelle Verunreinigungen abgeschabt werden.

Zur Entsorgung des Tablettenbruchs wird die Rinne 17 abgekippt (Fig. 6). Die Tablettenreste fallen in einen Restebehälter 23. Bei der Kippbewegung werden die Meß- bzw. Brechbacken 20 bzw. 24 mit dem an der Rinne 17 befestigten Schaber 19 von Tablettenresten gesäubert (Fig. 6). Die Rinne 17 wird dann in die Ruhe-Position I gekippt und für die nächste Messung bereitgestellt.

Bei den vorbekannten Einrichtungen zur Durchführung des Härteprüfverfahrens wird der Vorschub der beweglichen Brechbacke durch einen Schritt- bzw. Gleichstrommotor durchgeführt. Die hierbei auf die Tablette ausgeübte Kraft wird mit einer Meßzelle erfaßt. Bei einer sprunghaften Änderung des Kraftanstieges ergibt sich, daß die Tablette zerbrochen ist. Die Ansteuerung des Schritt- bzw. Gleichstrommotors erfolgt mit konstanter Spannung bzw. konstanter Frequenz. Sobald die Brechbacken die Tablette berühren, wird diese - abhängig von ihrer Härte - dem Vorschub des Antriebes der beweglichen Brechbacke einen entsprechenden Widerstand entgegensetzen. Die daraus resultierende Kraft-Zeit-Kennlinie ist also das Resultat der Tablettenhärte und des noch vorhandenen Drehmomentes des Antriebes. Nachteilig hierbei ist, daß diese Kombination zwischen Tablettenhärte und Drehmoment des Motors eine Kurve beschreibt, die nur für diese einzelne Tablette gültig ist. Eine reproduzierbare Messung ist daher nicht möglich.Durch die Streuung der unterschiedlichen Antriebe und der Ansteuerungen (auch bei Tablettenhärteprüfern gleichen Typs) ergeben sich unterschiedliche Kurven, mit der Folge,daß die Einrichtungen bzw. Geräte untereinander nur mit großen Toleranzen vergleichbar sind. Es wird also im Stand der Technik ein im Leerlauf konstanter Vorschub der beweglichen Brechbacke eingesetzt.

Erfindungsgemäß wird der konstante Vorschub der beweglichen Brechbacke 24 durch einen geschwindigkeitsgeregelten Vorschub ersetzt, der einen konstanten tablettenspetifisch wählbaren Kraftzuwachs je Zeiteinheit sichert. Anstatt des exponentiellen Kraftanstieges bei konstantem Vorschub der beweglichen Brechbacke 24 wird durch Anwendung einer Regelung ein linearer Vorschub der beweglichen Brechbacke 24 mit Kraftanstieg gewährleistet. Aufgabe dieser Regelung ist es, den Kraftanstieg je Zeiteinheit konstant zu halten. Dadurch ist die resultierende Krafteinleitung in die Tablette 2 zu jedem Zeitpunkt linear. Durch Verwendung der Regelung sind die Variablen "Tablettenhärte" und "Drehmoment des Motors" nicht mehr die bestimmenden Werte für den Kraftverlauf. Dies ist Voraussetzung für reproduzierbare Werte. Prinzipiell ist es unerheblich, welche Einheit die Regelung beim Zerstören der Tablette 2 konstant hält. Mögliche Verfahren sind: Konstanter Weg je Zeit oder konstanter Kraftanstieg je Zeit oder konstante Kraft je Weg.

Schließlich kalibrieren die vorbekannten Vorrichtungen bzw. Geräte ihre Druckmeßdose bzw. Meßbacke für den Härteprüfer, indem nach der Montage der Vorrichtung ein Gewicht aufgelegt wird. Dieses Verfahren birgt zwei wesentliche Nachteile:

Um die Kalibrierung vorzunehmen, wird ein Umbau an der Meßzelle vorgenommen, so daß diese sich nicht in derselben mechanischen Lage wie beim Tablettenprüfen befindet.Die Kalibrierung erfolgt statisch,die Belastung beim Bruch der Tablette ist hingegen dynamisch.

Diese beiden Verfahren sind nicht miteinander vereinbar, so daß sich durch die bisher angewandte Kalibrierung automatisch ein Meßfehler ergibt.

Die bei der Erfindung angewendete Kalibrierung ist dynamisch und arbeitet nach dem Vergleichsprinzip. Eine zweite Meßzelle wird anstelle der Tablette 2 zwischen die feste Brechbacke 24 und die bewegliche Brechbacke 20 eingebracht. Durch Vergleichsmessungen zwischen der eingebauten Meßbacke 24 und der nicht dargestellten Referenz-Meßzelle werden die Kalibrierung und die Bestimmung der Genauigkeit vorgenommen. Die Kalibrierung erfolgt bei Wahl unterschiedlicher Kraftanstiegsraten und Absolutwerte, d.h. der gesamte Meßbereich der Vorrichtung kann durchfahren werden. Die Kalibrierung bei konstantem Weg erfolgt auf ähnliche Weise. Anstelle der Meßzelle wird ein Wegaufnehmer eingebaut.

## Patentansprüche

1. Verfahren zur Kontrolle der Tablettenparameter Gewicht, Höhe und Härte mittels Wägen und Messen, vorzugsweise bei deren Herstellung auf Rundlauf-Tablettenpressen, zur dauerhaften Einhaltung vorgegebener Tablettenparameter,
**dadurch gekennzeichnet,**
**daß** wahlweise eine Summen- und/oder eine Einzelprüfung der Tabletten (2) durchgeführt wird, wobei bei der Summenprüfung eine definierte Anzahl von Tabletten (2) in einen Aufnahmebehälter (1) gebracht und zur Bestimmung ihres Summengewichtes auf eine Wägestation (3) transportiert und dort zusammen mit dem Aufnahmebehälter (1) gewogen und danach in ein Gefäß (4) entsorgt wird und bei der Einzelprüfung eine einzelne Tablette (2) in den Aufnahmebehälter (1) gebracht und zur Bestimmung ihres Einzelgewichts zu der Wägestation (3) transportiert und dort einzeln und ohne Aufnahmebehälter (1) gewogen und danach in das Gefäß (4) entsorgt wird oder die einzelne Tablette nach der Einzelgewichtsbestimmung zu den weiteren Prüfstationen (10,11) zur Bestimmung der Höhe und/oder der Härte vollautomatisch zu- und abgeführt wird.

2. Vorrichtung zur Durchführung des Verfahrens nach Anspruch 1, **dadurch gekennzeichnet, daß** zur Durchführung von Einzel- und/oder von Summengewichtsprüfungen der Aufnahmebehälter (1) für die Tabletten (2) über ein Transportsystem mit der Wägestation (3) verbunden ist und daß die Wägestation (3) und die Prüfstationen (10,11) zur Bestimmung der Tablettenhöhe und der Tablettenhärte einer einzelnen Tablette (2) untereinander über einen Saugheberarm (5) verbunden sind.

3. Vorrichtung nach Anspruch 2, **dadurch gekennzeichnet, daß** der Aufnahmebehälter (1) in seiner Längsachse geteilt und aufklappbar ist und im Bodenbereich zur Bodenmitte und damit zur Ausfallöffnung geneigte Schrägen (7) aufweist.

4. Vorrichtung nach Anspruch 2, **dadurch gekennzeichnet, daß** der Saugheberarm (5) einen Saugkopf (12) mit schwach konvex gekrümmtem Siebboden (13) aufweist, über welchen die Tabletten (2) bei strömender Saugluft (21) gehalten sind und wobei die Saugluft (21) den Tablettenabrieb (22) im Moment des Entstehens abführt.

5. Vorrichtung nach Anspruch 2, **dadurch gekennzeichnet, daß** die Wägestation (3) einen Wägeaufsatz (8) mit einem schwach nach unten konkav gekrümmten Siebeinsatz (14) und einen Wägeaufsatzboden (9) aufweist, in welchem Schlitze (15) angeordnet sind.

6. Vorrichtung nach Anspruch 2, **dadurch gekennzeichnet, daß** in Verbindung mit der Prüfstation (10) zur Höhenbestimmung eine Rüttelvorrichtung (16) vorgesehen ist, mit der die Tablette (2) vor der Höhenmessung in die stabile Schwerpunktlage gebracht ist.

7. Vorrichtung nach den Ansprüchen 2,3,4, **dadurch gekennzeichnet, daß** an der Prüfstation zur Bestimmung der Tablettenhärte ein Abstreifer (18) zur Vororientierung der Tabletten (2) vorgesehen ist und daß eine Rinne (17) mit der Querschnittsgeometrie einer geschwungenen Klammer oder mit einem Radius zwischen den Meß- und Brechbacken (20,24) vorgesehen ist, die zusammen mit dem Backenvorschub die Längs- und Mittenausrichtung der unterschiedlich geformten Tabletten (2) zur Härtebestimmung vornimmt.

8. Vorrichtung nach Anspruch 7, **dadurch gekennzeichnet, daß** an der Rinne (17) ein Schaber (19) zur Reinigung der Meß- und Brechbacken (20,24) der Härtemessung nach jedem Prüfvorgang vorgesehen ist.

9. Vorrichtung nach Anspruch 7 oder 8, **dadurch gekennzeichnet, daß** die Geschwindigkeit des Vorschubs der beweglichen Brechbacke (24) regelbar ist.

10. Vorrichtung nach einem der Ansprüche 7 bis 9, **dadurch gekennzeichnet, daß** die Kalibrierung dynamisch ist und nach dem Vergleichsprinzip arbeitet, wobei eine zweite Meßzelle anstelle der Tablette (2) in die Kalibriereinrichtung eingebracht wird und durch Vergleichsmessungen zwischen der eingebauten Meßzelle (24) und der zweiten Meßzelle (Referenz-Meßzelle) die Kalibrierung und die Bestimmung der Genauigkeit vorgenommen werden.

11. Vorrichtung nach den Ansprüchen 2 bis 10, **dadurch gekennzeichnet, daß** die Vorrichtungskomponenten (3,10,11) zur Prüfung des Gewichtes, der Höhe und der Härte einer Tablette (2) durch unabhängige Meßsysteme mit separater Justierung und Kalibrierung gebildet sind und daß die Vorrichtungskomponenten zur Steuerung, Bedienung und Anzeige ebenfalls Module bilden.

## Claims

1. Method for the inspection of the tablet parameters weight, height and hardness by means of weighing and measuring, preferentially during their production on rotary tablet presses, for the purpose of permanent adherence to prespecified tablet parameters,
wherein
a cumulative and/or single testing of the tablets (2) is selectively carried out where, for the cumulative testing, a defined number of tablets (2) is put into a container (1) and transported onto a weighing station (3) in order to determine their cumulative weight, at which location they are weighed together with the container (1) and, after this, are disposed of in a vessel (4) and, during a single testing, a single tablet (2) is put into the container (1) and transported to the weighing station (3) for the purpose of determining its single weight, and at this location it is individually weighed without the container (1), and then disposed of in the vessel (4), or the single tablet, after determining the single weight, is fully automatically led to and from the further testing stations (10, 11) for the purpose of determining the height and/or the hardness.

2. A device for implementing the method according to Claim 1, **wherein**, for the purpose of performing single and/or cumulative weight testing, the container (1) for the tablets (2) is connected by means of a transport system to the weighing station (3) and the weighing station (3) and the test stations (10, 11) are connected up among each other by way of a suction lifter arm (5) in order to determine the tablet height and the tablet hardness of one single tablet (2).

3. Device according to Claim 2, **wherein** the container (1) is split in its longitudinal axis and has the capability of opening out, and has inclined slants (7) in the bottom zone to the bottom centre, and subsequently to the discharge opening.

4. Device according to Claim 2, **wherein** the suction lifter arm (5) has a suction head (12) with a weak convex-curved sieve bottom (13), over which the tablets (2) are held at flowing suction air (21) and where the suction air (21) withdraws the tablet abrasive particles (22) at the moment of occurrence.

5. Device according to Claim 2, **wherein** the weighing station (3) has a weighing fixture (8) with a weak downward concave-curved sieve assembly (14) and a weighing fixture bottom (9), in which slots (15) are arranged.

6. Device according to Claim 2, **wherein**, in conjunction with the test station (10) and for the purpose of determining the height, a vibrating device (16) is envisaged with which the tablet (2) is brought into a stable gravitational point location for height measurement.

7. Device according to Claims 2, 3, 4 , **wherein**, at the test station for determining the tablet hardness, a wiper (18) is envisaged for preliminary orientation of the tablets (2), and a groove (17) with the cross-sectional geometry of a swung bracket or with a radius between the measurement and crushing jaws (20, 24) is envisaged which, together with the jaws forward thrust, carry out the longitudinal and central alignment of the differently shaped tablets (2) for the hardness determination.

8. Device according to Claim 7, **wherein**, at the groove (17), a scraper (19) is envisaged for the purpose of cleaning the measurement and crushing jaws (20, 24) of the hardness measurement after each test operation.

9. Device according to Claim 7 or 8, **wherein** the speed of the forward thrust of the mobile crushing jaw (24) is controllable.

10. Device according to one of the Claims 7 to 9, **wherein** the calibration is dynamic and works according to the principle of comparison, where a second measuring cell is brought into the calibrating device instead of the tablet (2) and, by means of comparative measurements between the installed measuring cell (24) and the second measuring cell (reference measuring cell), the calibration and the determination of the accuracy is carried out.

11. Device according to Claims 2 to 10, **wherein** the device components (3, 10, 11) are formed for the testing of the weight, the height and the hardness of a tablet (2) by means of independent measuring systems with separate adjustment and calibration, and the device components for regulation, control and indication also form modules.

## Revendications

1. Procédé pour contrôler les paramètres de comprimés, le poids, la hauteur et la dureté grâce au pesage et mesurage, de préférence au cours de leur fabrication sur des presses à comprimés rotatives, afin de maintenir perpétuellement des paramètres de comprimés prédéterminés, **caractérisé en ce que**, au choix, on vérifie le poids totalisé et / ou unitaire des comprimés (2); un nombre défini de comprimés (2) étant introduit dans un récipient de réception (1) lors de la vérification des poids totalisés et lesdits comprimés étant transportés pour déterminer leurs poids totalisés sur une station de pesage (3) et y être pesés ensemble avec le récipient de réception (1) puis introduits dans un récipient (4) et **caractérisé en ce que** l'on introduit au cours de la vérification individuelle un seul comprimé (2) dans le récipient de réception (1) et **en ce que** l'on transporte ledit comprimé vers la station de pesage (3) pour y être pesé individuellement et sans le récipient de réception (1) et **en ce que** l'on introduit ensuite ledit comprimé dans le récipient (4) ou **caractérisé en ce que** les comprimés individuels sont amenés de façon complètement automatique vers les autres stations de vérification (10, 11) après la détermination des poids unitaires afin de déterminer la hauteur et / ou la dureté.

2. Dispositif pour réaliser le procédé selon la revendication 1, **caractérisé en ce que** le récipient de réception (1) pour les comprimés (2) est relié, pour vérifier les poids unitaires et / ou totalisés, par un système de transport à la station de pesage (3) et **caractérisé en ce que** la station de pesage (3) et les stations de vérification (10, 11) pour déterminer la hauteur et la dureté d'un seul comprimé (2) sont liées entre elles par un bras de levage et d'aspiration (5).

3. Dispositif selon la revendication 2, **caractérisé en ce que** le récipient de réception (1) est divisé et susceptible d'être rabattu sur son axe longitudinal et comporte dans la zone de fond vers le milieu de fond et donc vers l'ouverture de sortie, des surfaces inclinées (7).

4. Dispositif selon la revendication 2, **caractérisé en ce que** le bras de levage et d'aspiration (5) comporte une tête d'aspiration (12) avec un fond à tamis (13) légèrement courbé et convexe sur lequel fond les comprimés (2) sont maintenus grâce à l'air courant aspiré et l'air aspiré (21) éliminé lors de leurs création les débris des comprimés (22).

5. Dispositif selon la revendication 2, **caractérisé en ce que** la station de pesage (3) comporte un accessoire de pesage (8) avec un insert à tamis (14) légèrement courbé vers le bas de façon concave et un fond d'accessoire de pesage (9) dans lequel sont disposées des fentes (15).

6. Dispositif selon la revendication 2, **caractérisé en ce qu'**un dispositif pour secouer est prévu en liaison avec la station de pesage (10) pour déterminer la hauteur grâce auquel le comprimé (2) est placé dans une position stable du point de gravité avant de mesurer la hauteur.

7. Dispositif selon les revendications 2, 3, 4, **caractérisé en ce qu'**il est prévu sur la station de vérification afin de déterminer la dureté des comprimés, un racleur (18) pour pré-orienter les comprimés (2) et **en ce qu'** on a prévu une gorge (17) dont la géométrie en section transversale a la forme d'une pince arquée ou présente un rayon entre les joues de mesurage et de coupe (20, 24); laquelle gorge oriente, au fur et à mesure que la joue avance, les comprimés (2) de formes différentes de manière longitudinale et médiane pour déterminer leurs duretés.

8. Dispositif selon la revendication 7, **caractérisé en ce que** sur une gorge (17) on a prévu un racleur (19) pour nettoyer après chaque vérification les joues de mesurage et de coupe (20, 24) destinées au mesurage de la dureté.

9. Dispositif selon la revendication 7 ou 8, **caractérisé en ce que** la vitesse de l'avancement de la joue de coupe (24) mobile est réglable .

10. Dispositif selon une des revendications 7 à 9, **caractérisé en ce que** le calibrage est dynamique et fonctionne selon le principe de comparaison ; une deuxième cellule de mesure étant introduite à la place des comprimés (2) dans le dispositif de calibrage et **caractérisé en ce que** le calibrage et la détermination de la précision sont effectués par des mesures de comparaison entre la cellule de mesure (24) implantée et la deuxième cellule de mesure (cellule de mesure de référence).

11. Dispositif selon les revendications 2 à 10, **caractérisé en ce que** les composants du dispositif (3, 10, 11) sont formés à partir de systèmes de mesure indépendants avec une justification et un calibrage séparés afin de vérifier le poids, la hauteur et la dureté d'un comprimé (2) et **en ce que** les composants de pilotage, de commande et d'affichage du dispositif forment également des modules.
